# EUROPÄISCHE PATENTANMELDUNG

(11) **EP 3 868 840 A1**
(43) Veröffentlichungstag der Anmeldung: **25.08.2021**
(21) Anmeldenummer: 21157055.1
(22) Anmeldetag: 15.02.2021
(51) Int. Cl.: C09D 5/14, A61L 2/08, A61L 2/10, C09D 5/22, C09K 11/77

(54) **ZUSAMMENSETZUNG ZUR HERSTELLUNG VON BESCHICHTUNGEN MIT ANTIMIKROBIELLER EIGENSCHAFT**

(30) Priorität: 18.02.2020 EP 20157842
(71) Anmelder: Evonik Operations GmbH, 45128 Essen (DE)
(72) Erfinder: Schulte, Simone, 45133 Essen (DE); Hallack, Markus, 46514 Schermbeck (DE); Krusenbaum, Sabine, 45134 Essen (DE); Janke, Christina, 45327 Essen (DE)
(74) Vertreter: Evonik Patent Association

(57) **Zusammenfassung**

Die Erfindung betrifft eine härtbare Zusammensetzung zur Herstellung von Beschichtungen mit anti-mikrobieller Eigenschaft enthaltend
- mindestens ein filmbildendes Polymer,
- mindestens einen up-conversion Leuchtstoff,
- optional mindestens ein Additiv,
- optional mindestens einen Härter,
wobei der Leuchtstoff ausgewählt ist aus der idealisierten allgemeinen Formel (I)

A_{1-x-y-z}B*_{y}B₂SiO₄:Ln¹ₓLn²_{z,} I

mit
x = 0,0001 - 0,05, z = 0 oder z = 0,0001 bis 0,3 und y = x + z,
A ausgewählt aus Mg, Ca, Sr und Ba,
B ausgewählt aus Li, Na, K, Rb und Cs,
B* ausgewählt aus Li, Na und K, wobei B gleich B* oder B ungleich B* ist, bevorzugt B und B* sind ungleich, und
Ln¹ ausgewählt aus Praseodym (Pr), Erbium (Er) und Neodym (Nd),
optional Ln² ausgewählt aus Gadolinium (Gd).

## Beschreibung

Die Erfindung betrifft eine härtbare Zusammensetzung zur Herstellung von Beschichtungen mit anti-mikrobieller Eigenschaft, deren Verwendung und daraus hergestellte Beschichtungen und Gegenstände, die damit beschichtet sind.

Tagtäglich sind Menschen Millionen von Mikroorganismen wie Bakterien, Pilzen und Viren ausgesetzt. Viele dieser Mikroorganismen sind nützlich bzw. sogar notwendig. Dennoch gibt es neben den harmloseren Vertretern auch krankheitsverursachende oder sogar tödliche Bakterien, Pilze und Viren.

Durch den täglichen Umgang mit anderen Menschen und den Kontakt mit Gegenständen, die andere benutzt haben, können Mikroorganismen übertragen werden. Die antimikrobielle Ausstattung von Oberflächen erfolgt insbesondere in hygienesensiblen Bereichen. Anwendungsbereiche sind vor allem Oberflächen von medizintechnischen Geräten und Bedarfsgegenstände in Krankenhäusern sowie in Einrichtungen des ambulanten Gesundheits- und Sozialwesens. Hinzu kommen Oberflächen im öffentlichen Raum, im Lebensmittelsektor und in der Tierhaltung. Die Verbreitung von pathogenen Mikroorganismen stellt heute ein großes Problem im Pflegebereich und in der Medizin dar, und auch überall dort, wo viele Menschen auf engem Raum verkehren. Ein besonders Risiko stellt gegenwärtig auch das vermehrte Aufkommen sogenannter multiresistenter Keime dar, die unempfindlich gegen übliche Antibiotika geworden sind.

Um das Risiko der Verbreitung von pathogenen Erregern über Berührungsoberflächen zu verringern, werden, neben Maßnahmen zur Standardhygiene, antimikrobielle Technologien und Werkstoffe genutzt. Chemische Substanzen oder die Verwendung physikalischer Verfahren können den Vermehrungsprozess von Mikroorganismen kritisch beeinflussen. Zu den physikalischen Methoden zählen z. B. Hitze, Kälte, Strahlung oder Ultraschall, etc. Bei den chemischen Methoden sind Halogene, Metallionen, organische Verbindungen und Farbstoffe, giftige Gase, etc. bekannt.

Obwohl in den meisten Fällen chemische und physikalische Methoden außerordentlich effektiv bei der Zerstörung von Mikroorganismen sind, so haben sie doch nur einen kurzzeitigen Effekt, fördern die Entstehung von Resistenzen und sind unter Umständen für manche Anwendungen nicht geeignet, da sie zur Zerstörung der zu schützenden Oberflächen führen. Den größten Nachteil stellt allerdings, gerade bei chemischen, organischen Substanzen, die Gefährlichkeit bzw. Giftigkeit für den Menschen dar. Bestimmte Substanzen, wie z. B. Formaldehyd, welches viele Jahre als Desinfektionsmittel Anwendung fand, stehen inzwischen im Verdacht, Krebs zu verursachen oder extrem umweltschädlich zu sein.

Oberflächen mit antimikrobieller Wirkung könnten einen entscheidenden Beitrag zur Lösung dieser Probleme leisten. Die heute gängigen Verfahren zur Erzeugung solcher antimikrobiellen Eigenschaften verwenden überwiegend in das Material eingearbeitete Wirkstoffe wie z.B. Silberpartikel, Kupferpartikel, deren Metalloxide oder quaternäre Ammoniumverbindungen. Dabei werden die antimikrobiell wirkenden Metalle, Metalloxide oder Metalloxidgemische häufig zu Nanoteilchen verarbeitet und dann Farben, Lacken oder Polymerwerkstoffen zugemischt. Der breite Einsatz von Metallpartikeln ist in Frage zu stellen, da die langfristige Wirkung dieses Schwermetalls auf Mensch und Umwelt kaum abzuschätzen ist.

Beispielsweise offenbart die WO 2019/197076 Partikel, die mit einer Schicht ausgestattet sind, die sowohl Antimon-Zinnoxid als auch Manganoxid enthält. Dem Fachmann ist bekannt, dass die antimikrobiellen Oberflächen aufgrund des elektrochemischen Verhaltens von Metallen erzeugt werden, die bei Anwesenheit von Feuchtigkeit mikrogalvanische Zellen und durch die mikroelektrischen Felder keimabtötende Wirkung entfalten.

Es ist ebenso bekannt, UV-Strahlung in der Medizin oder in der Hygiene einzusetzen, um beispielsweise Wasser, Gase oder Oberflächen zu desinfizieren. So wird in der Trinkwasseraufbereitung seit langem UV-Strahlung zur Reduzierung der Anzahl von fakultativ krankheitserregenden Mikroorganismen im Wasser eingesetzt. Dabei wird vorzugsweise UV-C-Strahlung im Wellenlängenbereich zwischen 100 nm und 280 nm eingesetzt. Der Einsatz elektromagnetischer Strahlung mit unterschiedlichen Wellenlängen sollte die unterschiedliche Absorption der verschiedenen Proteine, den in Mikroorganismen, Geweben oder Zellen enthaltenden Amino-/Nukleinsäuren (z.B. DNA) sowie Peptidbindungen zwischen den einzelnen Säuren berücksichtigen. So absorbiert DNA elektromagnetische Strahlung innerhalb des Wellenlängenbereichs zwischen 200 nm und 300 nm gut und zwischen 250 nm und 280 nm besonders gut, so dass diese Strahlung gegen DNA besonders geeignet ist. Es können also krankheitserregende Mikroorganismen (Viren, Bakterien, Hefen, Schimmelpilze u.a.) mit einer solchen Bestrahlung inaktiviert werden. Je nach Dauer und Intensität der Bestrahlung kann die Struktur von DNA zerstört werden. Somit werden stoffwechselaktive Zellen inaktiviert und/oder deren Vermehrungsvermögen kann beseitigt werden. Vorteilhaft bei der Bestrahlung mit UV-Licht ist, dass die Mikroorganismen keine Resistenz dagegen entwickeln können.

Des Weiteren ist es neben einer direkten Bestrahlung mit elektromagnetischer Strahlung aus dem Wellenlängenbereich des UV-Lichts auch bekannt, den Effekt der so genannten up-conversion auszunutzen. Dabei werden Leuchtstoffpartikel eingesetzt, mit denen elektromagnetische Strahlung mit Wellenlängen oberhalb des UV-Lichts, insbesondere sichtbares Licht oder Infrarotlicht, in elektromagnetische Strahlung mit geringerer Wellenlänge gewandelt werden kann, so dass die Emission von in gewünschter Form wirkender Strahlung von den einzelnen Leuchtstoffpartikeln erreicht werden kann.

DE 10 2015 102 427 betrifft einen elektromagnetische Strahlung im Wellenlängenbereich des UV-Lichts emittierenden Körper. Bei dem Körper sind in einem oberflächennahen Bereich innerhalb des Werkstoffs, aus dem der Körper gebildet ist oder einer Beschichtung auf dem Körper, Leuchtstoffpartikel eingebettet. Hierbei wird nur allgemein angegeben, dass die Leuchtstoffpartikel einer auf dem Werkstoff auszubildenden Beschichtung direkt bei der Verarbeitung zugegeben werden, dabei sollte der jeweilige Werkstoff eine geeignete Konsistenz bzw. Viskosität aufweisen. Hinsichtlich geeigneter Polymere und Additive schweigt DE 10 2015 102 427.

US 2009/0130169 A1 bzw. WO 2009/064845 A2 beschreibt Leuchtstoffe, die in Polyvinylchloride, Acrylbutadiene, Olefine, Polycarbonate, Styrole oder Nylon eingebracht werden können, welche durch die up-conversion Eigenschaft der Leuchtstoffe pathogene Mikroorganismen abtöten. Es handelt sich hierbei um Leuchtstoffe, die bei einer Temperatur von 1800°C - 2900°C hergestellt werden. Darüber hinaus offenbart US 2009/0130169 A1 eine flüssige Zusammensetzung enthaltend ein Polyurethan, ein Acrylatpolymer und Füllstoffe, optional einen Vernetzer. US 2009/0130169 A1 geht auf die antimikrobielle Wirkung der Leuchtstoffe ein, jedoch werden die Verträglichkeit der Komponente in der Beschichtungszusammensetzung oder die Eigenschaften der Beschichtungsoberflächen, wie etwa die Lackoberflächen, nicht thematisiert. Das Erscheinungsbild von Beschichtungsoberflächen spielen jedoch beim Anwender eine besondere Rolle.

Die US 2009/0130169 A1 bzw. WO 2009/064845 A2 offenbart zwar eine Zusammensetzung enthaltend besagter Leuchtstoffe mit behaupter antimikrobieller Wirkung, zeigt jedoch weder einen Nachweis der up-conversion Eigenschaft noch mikrobiologischer Untersuchungen. Das darin offenbarte Verfahren führt zu keinem Leuchtstoff, der eine up-conversion Eigenschaft aufweist, sondern zu einem amorphen und glasartigen Produkt.

Die Anforderungen an Lacke und Farben sind vielfältig. Grundsätzlich haben Lack- bzw. Farbbeschichtungen zwei Aufgaben bzw. Funktionen, die schützende und die dekorative Funktion. Sollten im Weiteren nur der Begriff "Lackbeschichtung" aufgeführt sein, so sind beide Arten der Beschichtung gemeint. Sie verschönern, schützen und bewahren Materialien wie Holz, Metall oder Kunststoff. Demnach werden auf der einen Seite brillante und glatte Lackschichten gefordert, auf der anderen Seite eine geschlossene Lackschicht zur Sicherstellung der chemischen und mechanischen Beständigkeit, eine gewisse Gleitfähigkeit der Lacke oder eine besondere Haptik.

Dementsprechend stellt sich die Erfindung die Aufgabe, eine härtbare Zusammensetzung der eingangs genannten Art zur Verfügung zu stellen, mit der Beschichtungen hergestellt werden können, bei denen ein langandauernder Schutz gegen Mikroorganismen bereitgestellt wird, und ohne dass die übrigen Eigenschaften, insbesondere die Lagerstabilität, signifikant beeinträchtigt werden.

Zur Lösung der Aufgabe wird daher eine Härtbare Zusammensetzung zur Herstellung von Beschichtungen mit anti-mikrobieller Eigenschaft enthaltend
- mindestens ein filmbildendes Polymer,
- mindestens einen up-conversion Leuchtstoff,
- optional mindestens ein Additiv,
- optional mindestens einen Härter,
wobei der Leuchtstoff ausgewählt ist aus der idealisierten allgemeinen Formel (I)

A_{1-x-y-z}B*_{y}B₂SiO₄:Ln¹ₓLn²_{z}, (I)

mit
x = 0,0001 - 0,05, z = 0 oder z = 0,0001 bis 0,3 und y = x + z,
A ausgewählt aus Mg, Ca, Sr und Ba,
B ausgewählt aus Li, Na, K, Rb und Cs,
B* ausgewählt aus Li, Na und K, wobei B gleich B* oder B ungleich B* ist, bevorzugt B und B* sind ungleich, und
Ln¹ ausgewählt aus Praseodym (Pr), Erbium (Er) und Neodym (Nd),
optional Ln² ausgewählt aus Gadolinium (Gd),
   vorgeschlagen.

Überraschend wurde nun gefunden, dass mit der erfindungsgemäßen Zusammensetzung Beschichtungen hergestellt werden konnten, die eine antimikrobielle Wirkung und keine Beeinträchtigung des Oberflächenbildes aufweisen.

Vorzugsweise ist der Leuchtstoff mit Praseodym dotiert, der in der erfindungsgemäßen Zusammensetzung verwendet wird.

Bevorzugt für die erfindungsgemäße Zusammensetzung ist der Leuchtstoff mit Praseodym dotiert und mit Gadolinium co-dotiert.

Vorzugsweise ist der Leuchtstoff eine erstarrte Schmelze aus kristallinen Silikaten oder aus kristallinen Silikaten, dotiert mit Lanthanoid-Ionen, umfassend mindestens einen Alkalimetallion und mindestens einen Erdalkalimetallion.

Bevorzugt für die erfindungsgemäße Zusammensetzung ist der Leuchtstoff ausgewählt aus der idealisierten allgemeinen Formel (la)

A_{1-x-y-z}B*_{y}B₂SiO₄:Prₓ,Gd_{z}, **(** **Ia)**

mit A = Mg, Ca, Sr, Ba und B = Li, Na, K, Rb, Cs, und,
wobei in Formel Ia x = 0,0001 - 0,05, z = 0 oder z = 0,0001 bis 0,3 und y = x + z ist,
B* ausgewählt aus Li, Na und K, die zur Ladungsausgleich der Silikate dienen,
wobei B gleich B* oder B ungleich B* ist, bevorzugt B und B* sind ungleich.

Vorzugsweise wird für die erfindungsgemäße Zusammensetzung der Leuchtstoff ausgewählt aus der allgemeinen Formel (II)

(Ca₁₋ₐSrₐ)_{1-2b}Ln_{b}Na_{b}Li₂SiO₄ **II**

wobei
a = 0,0001 bis 1, bevorzugt 0,0001 bis 0,1
b = 0,0001 bis 1, bevorzugt 0,0001 bis 0.1
und Ln = Lanthanoid-Ion ausgewählt aus Praseodym, Gadolinium, Erbium, Neodym und für die Co-Dotierung mindestens einen von denen, bevorzugt I Gadolinium.

Es sei hier anzumerken, dass die für die vorliegende Erfindung erforderlichen Leuchtstoffe aus der bisher unveröffentlichten europäischen Patentanmeldung mit dem Anmeldeaktenzeichen EP 19202910.6 offenbart sind.

Bevorzugt handelt es sich bei dem Leuchtstoff um einen, der bei Bestrahlung mit elektromagnetischer Strahlung mit geringerer Energie und höherer Wellenlänge im Bereich von 2000 nm bis 400 nm, insbesondere im Bereich von 800 nm bis 400 nm, elektromagnetische Strahlung mit höherer Energie und kürzerer Wellenlänge im Bereich von 400 nm bis 100 nm, bevorzugt im Bereich von 300 nm bis 200 nm emittiert, wobei die Intensität des Emissionsmaximums der elektromagnetischen Strahlung mit höherer Energie und kürzerer Wellenlänge eine Intensität von mindestens 1 • 10³ counts/(mm²*s), bevorzugt höher als 1 • 10⁴ counts/(mm²*s), besonders bevorzugt höher als 1 • 10⁵ counts/(mm²*s). Die Emissionsspektra werden angeregt mittels eines Lasers, insbesondere eines Lasers mit einer Leistung von 75mW bei 445 nm und/oder einer Leistung von 150 mW bei 488 nm.

Vorzugsweise weist der Leuchtstoff gemäß Formel (II) XRPD Signale im Bereich von 23° 2Θ to 27° 2Θ und von 34° 2Θ bis 39.5° 2Θ auf, wobei die Signale mittels der Bragg-Brentano Geometrie und der Cu-Ka Radiation bestimmt werden. Einzelheiten der Messmethode können aus der noch unveröffentlichten europäischen Patentanmeldungen EP 19202910.6 entnommen werden.

Die bisher unveröffentlichten europäische Patentanmeldung EP 19202910.6 widmet sich der Herstellung von Leuchtstoffen, insbesondere von Leuchtstoffen gemäß Formel (I), Formel (la) und Formel (II). Darin wird ein Verfahren beschrieben, umfassend folgende Schritte:
- i) Bereitstellung mindestens eines Lanthanoidsalzes, ausgewählt aus Lanthanoidnitrate, Lanthanoidcarbonate, Lanthanoidcarboxylate, bevorzugt Lanthanoidacetate, Lanthanoidsulphate, Lanthanoidoxide, besonders bevorzugt Pr₆O₁₁ und/oder Gd₂O₃, wobei die Lanthanoidion in den Lanthanoidoxiden oder Lanthanoidsalzen ausgewählt ist aus Praseodymium, Gadolinium, Erbium, Neodym und für die Co-Dotierung mindestens zwei davon,
- ii) Bereitstellung eines Silikats, bevorzugt eines Silikatsalzes, besonders bevorzugt eines Alkalimetallsalzes des Silikats,
- iii) Bereitstellung mindestens eines Erdalkylimetallsalzes und mindestens eines Alkalimetallsalzes, bevorzugt eines Alkalimetallsilikats ausgewählt aus einem Lithiumsalz oder einer Lithiumverbindung und optional ausgewählt aus einem Natriumsalz und Kaliumsalz, vorzugsweise das Salz des Lithiumsalzes und ist ein Lithiumsilikat,
   - a) Mischen i), ii) und iii) mittels Mahlens zur Erhaltung einer Mischung, oder
   - b) Mischen i), ii) und iii) in einem organischen polaren oder unpolaren Lösemittel, das kein protisches Lösemittel ist, zur Erhaltung einer Mischung; die Mischung aus b) wird kalziniert (Schritt 1a) bei 600 °C bis 1000 °C zur Entfernung der organischen Komponente, bevorzugt wird die Kalzinierung bei 600 °C bis 1000 °C für mindestens 1 h durchgeführt, vorzugsweise mehr oder gleich 2h, unter normaler (Luft)Atmosphäre zur Erhaltung einer kalzinierten Mischung,
      - Kalzinieren der Mischung aus a) oder der kalzinierten Mischung aus b) in einem Kalzinierungsschritt, bevorzugt unter Luft bei einer Temperatur unterhalb der Schmelztemperatur des silikatbasierten Materials, wobei mindestens eine partielle Kristallisation stattfindet, bevorzugt in einem weiteren Kalzinierungsschritt (Schritt 1b) bei einer Temperatur von 50 bis 200°C für mindestens 3h, vorzugsweise unter Luft, unterhalb der Schmelztemperatur des silikatsbasierten Materials, um das silikatbasierte Material zu kristallisieren, bevorzugt bei einer Temperatur von 800 bis 900 °C, besonders bevorzugt bei ungefähr 850 °C, für mindestens 3 h, vorzugsweise für mindestens 12 h, vorzugweise unter Luft,
      - in einem weiteren Kalzinierungsschritt bei steigender Temperatur, vorzugsweise über 800°C und 50 bis 200°C unterhalb des Schmelzpunktes (Schritt 2) des Materials, z.B. bei 850°C für mindestens 3h, besonders bevorzugt für mindestens 6h, unter reduzierter Atmosphäre, dabei werden die Lanthanoide zu Ln³⁺ Ions reduziert,
      - Erhalten eines silikatbasierten Lanthanoid-Ion dotierten Materials, bevorzugt nach Abkühlen des Materials.

Weitere detaillierte Ausführungsformen des Verfahrens können aus der EP 19202910.6 entnommen werden.

Es hat sich überraschend herausgestellt, dass die Leuchtstoffe gemäß der EP 19202910.6 die erforderlichen up-conversion Eigenschaften aufweisen, die für die antimikrobielle Wirkung verantwortlich sind. D. h. diese Leuchtstoffe können elektromagnetische Strahlung mit Wellenlängen oberhalb des UV-Lichts, insbesondere sichtbares Licht oder Infrarotlicht, in elektromagnetische Strahlung mit geringerer Wellenlänge emittieren und zwar in dem Bereich, bei dem z.B. die DNA der Mikroorganismen zerstört werden kann. Demnach sind diese Leuchtstoffe für die erfindungsgemäße Zusammensetzung sehr gut geeignet.

Es ist auch vorstellbar, den erfindungsgemäßen Leuchtstoff wie folgt herzustellen:
Als Ausgangsstoffe werden CaCO₃ (Fa. Alfa Aesar, 99.5%), Li₂CO₃ (Fa. Alfa Aesar, 99%), SiO₂ (Aerosil 200, Evonik), Pr₆O₁₁ (Treibacher, 99.99%), and Na₂CO₃ (Merck, 99.9%) verwendet. Eine stöchiometrische Mischung dieser Verbindungen wird in Aceton für 30 Minuten vermischt. Nachdem das Aceton bei Raumtemperatur vollständig verdunstet ist, wird die Mischung in einem Korundtiegel überführt. Die Mischung wird zweimal kalziniert. Die erste Kalzinierung wird in einem Schmelzofen bei 850°C für 12h unter Luftzufuhr und die zweite Kalzinierung bei 850°C für 6h unter 95/5 N₂/H₂ durchgeführt. Das Endprodukt wird in einer Achatreibschale anschließend gemahlen.

Eine weitere Aufgabe der Erfindung ist die Auswahl filmbildender Polymere, die für die härtbare Zusammensetzung mit antimikrobieller Eigenschaft eingesetzt werden können. Grundsätzlich kommen alle aus dem Stand der Technik bekannten filmbildenden Polymere in Frage.

Vorzugsweise weist das filmbildende Polymer funktionelle Gruppen, vorzugsweise acide Wasserstoffe, auf, welche mit einem isocyanathaltigen Härter reaktiv sind und ggf. mit einem Katalysator katalysiert wird.

Vorteilhafterweise ist das filmbildende Polymer ausgewählt aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Asparticpolymer oder Polyesterpolymere, das mit einem isocyanathaltigen Härter reagiert.

Vorzugsweise ist das filmbildende Polymer resonanzarm.

Dem Fachmann sind die physikalischen Wechselwirkungen an der Oberfläche bekannt. Je nach Material und dessen Materialoberfläche treten bei Lichteinfall eine Vielzahl von Effekten an der Oberfläche auf. Das auftreffende Licht wird zum Teil absorbiert, ein Teil wird reflektiert und je nach Materialoberfläche auch gestreut. Licht kann auch erst absorbiert und dann wieder emittiert werden. Bei opaken, halbdurchlässigen oder lichtdurchlässigen Materialien kann das Licht auch durch den Körper durchdringen (Transmission). In einigen Fällen wird das Licht an der Oberfläche sogar polarisiert oder gebeugt. Manche Objekte können sogar Licht emittieren (beleuchtete Anzeigen, LED-Segmente, Displays), in einer anderen Lichtfarbe fluoreszieren oder phosphoreszieren (nachleuchten).

Resonanzarm bedeutet im Sinne dieser Erfindung, dass das filmbildende Polymer geringe Absorption, Reflexion, Remission und Streuung aufweist. Dagegen soll vorzugsweise die Transmission ausgeprägt sein.

Es konnte nämlich überraschend festgestellt werden, dass die erfindungsgemäße filmbildende Polymere, die resonanzarm sind, eine verbesserte antimikrobielle Wirkung aufweisen, bedingt dadurch, dass mehr elektromagnetische Strahlung mit geringerer Energie und höherer Wellenlänge im Bereich von 2000 nm bis 400 nm, insbesondere im Bereich von 800 nm bis 400 nm, transmittiert wird und resultierend daraus mehr elektromagnetische Strahlung mit höherer Energie und kürzerer Wellenlänge im Bereich von 400 nm bis 100 nm, bevorzugt im Bereich von 300 nm bis 200 nm, emittieren kann.

Es wurde festgestellt, dass je höher die Transmission ist, desto höher ist auch die Emission, die für die antimikrobielle Wirkung ausschlaggebend ist.

Vorzugsweise liegt die Transmission des filmbildenden Polymers bei mindestens 75%, bevorzugt mindestens 80% und besonders bevorzugt mindestens 85%, gemessen bei einer Wellenlänge von 260 nm.

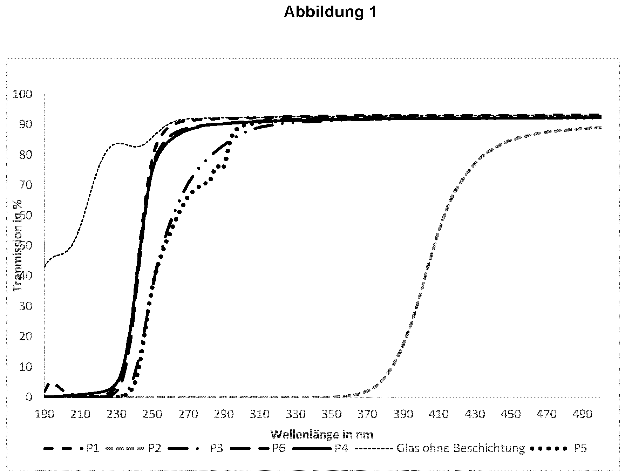

Vorzugsweise liegt die Transmission des filmbildenden Polymers bei mindestens 75%, bevorzugt mindestens 80% und besonders bevorzugt mindestens 85%, gemessen bei einer Wellenlänge von 500 nm.
Zur Verdeutlichung sei hier angemerkt, dass die Transmission bei einer anderen Wellenlänge definiert werden kann, siehe Abbildung 1. Für die vorliegende Erfindung wurden die Wellenlängen 260 nm beispielshaft für die emittierte Wellenlänge und 500 nm beispielhaft für die Anregungswellenlänge gewählt, die zum einen für die up-conversion und zum anderen signifikant für die antimikrobielle Wirkung verantwortlich sind.

Bei einer Transmission von 100% beispielsweise, gemessen bei einer Wellenlänge 260 nm, wird die gleiche Menge an Strahlung umgewandelt und emittiert, d. h. es gibt keine Verluste durch Absorption, Streuungen oder dergleichen. Bei einer Transmission von 80%, gemessen bei einer Wellenlänge 260 nm, transmittiert 20% nicht, vermutlich aufgrund von Absorption, Reflexion, Remission und/oder Streuung. Demzufolge kann nur 80% der Strahlung der Wellenlänge 260 nm emittiert werden.

Diese bedeutende Erkenntnis ist wichtig bei der Auswahl der filmbildende Polymere. Polymeren, die z.B. 0% Transmission aufweisen, eignen sich nicht für die erfindungsgemäße härtbare Zusammensetzung. Sie transmittieren keine elektromagnetische Strahlung mit geringerer Energie und höherer Wellenlänge und demzufolge können in der Zusammensetzung enthaltene Leuchtstoffe diese elektromagnetische Strahlung nicht in elektromagnetische Strahlung mit höherer Energie und kürzerer Wellenlänge umwandeln und emittieren, die für die antimikrobielle Wirkung erforderlich ist.

Vorzugsweise weist die erfindungsgemäße Zusammensetzung eine Transmission bei mindestens 75%, bevorzugt bei mindestens 80% und besonders bevorzugt bei mindestens 85% gemessen bei 260 nm beträgt.

Vorzugsweise weist die erfindungsgemäße Zusammensetzung eine Transmission bei mindestens 75%, bevorzugt bei mindestens 80% und besonders bevorzugt bei mindestens 85% gemessen bei 500 nm beträgt.

Die Transmissionen werden vorzugsweise mit einem "Specord 200 Plus" UV/VIS-Zweistrahlspektrometer der Firma Analytik Jena gemessen. Mit einem Holmiumoxidfilter erfolgt eine interne Wellenlängenkalibrierung. Die Proben werden mit monochromatisiertem Licht einer Deuterium- (UV-Bereich) oder einer Wolfram-Halogen-Lampe (sichtbarer Bereich) durchstrahlt. Die spektrale Bandbreite beträgt 1,4 nm. Das monochromatisierte Licht wird in einen Mess- und einen Referenzkanal aufgeteilt und ermöglicht das direkte Messen gegen eine Referenzprobe. Die durch die Probe transmittierende Strahlung wird von einer Photodiode detektiert und verarbeitet.

Es ist vorstellbar, eine Zusammensetzung mit einer geringen Transmission von unter 70 % einzusetzen; sie wirken möglicherweise auch noch antimikrobiell, jedoch ist der Wirkungsgrad sehr moderat.

Vorzugsweise weisen die Leuchtstoffe eine durchschnittliche Partikelgröße von d50 von 0,1 - 100 µm, bevorzugt d50 = 1 - 50 µm, gemessen nach ISO 13320:2020 und USP 429, beispielsweise mit einem Gerät der Fa. Horiba, LA-950 Laser Particle Size Analyzer, auf. Um die Leuchtstoffe in der erfindungsgemäßen Zusammensetzung gut einzubinden und/oder zu stabilisieren, können vorzugsweise verschiedene Additive zugesetzt werden.

Bevorzugt sind die Additive ausgewählt aus der Gruppe der Dispergiermittel, Rheologiehilfsmittel, Verlaufsmittel, Netzmittel, Entschäumer und UV-Stabilisierungsmittel.

Es wurde überraschend festgestellt, dass durch etwaigen Zusatz an Additiven in die erfindungsgemäße Zusammensetzung die Transmission reduziert wird.

Demnach weist die erfindungsgemäße Zusammensetzung in einer weiteren Ausführungsform, in der Additive eingesetzt werden, vorzugsweise eine Transmission bei mindestens 70%, bevorzugt bei mindestens 75% und besonders bevorzugt bei mindestens 80% gemessen bei 260 nm beträgt.

Demnach weist die erfindungsgemäße Zusammensetzung in einer weiteren Ausführungsform, in der Additive eingesetzt werden, vorzugsweise eine Transmission bei mindestens 70%, bevorzugt bei mindestens 75% und besonders bevorzugt bei mindestens 80% gemessen bei 500 nm beträgt.

Vorzugsweise weist die erfindungsgemäße Zusammensetzung einen Härter auf, der ausgewählt ist aus der Gruppe der aliphatischen oder cycloaliphatischen Isocyanate.

Beispiele für isocyanathaltige Härter sind monomere Isocyanate, polymere Isocyanate und Isocyanat-Prepolymere. Polyisocyanate werden gegenüber monomeren Isocyanaten aufgrund ihrer geringeren Toxizität bevorzugt. Beispiele für Polyisocyanate sind Isocyanurate, Uretdione und Buirete basierend auf Diphenylmethan Diisocyanat (MDI), Toluol Diisocyanat (TDI), Hexamethylen Diisocyanate (HDI) and Isophoron Diisocyanat (IPDI). Beispiele für kommerziell erhältliche Produkte sind unter dem Markennamen DESMODUR® von Covestro oder VESTANAT von der Evonik Industries. Bekannte Produkte sind DESMODUR® N3400, DESMODUR® N3300, DESMODUR® N3600 DESMODUR® N75, DESMODUR® XP2580, DESMODUR® Z4470, DESMODUR® XP2565 and DESMODUR® VL, von Covestro. Weitere Beispiele sind VESTANAT® HAT 2500 LV, VESTANAT® HB 2640 LV oder VESTANAT® T 1890E von der Evonik Industries. Beispiele für Isocyanat-Prepolymere sind DESMODUR® E XP 2863, DESMODUR® XP 2599 oder DESMODUR® XP 2406 von Covestro. Weitere dem Fachmann bekannte Isocyanat-Prepolymere können eingesetzt werden.

Es ist vorstellbar, Katalysatoren zur Aushärtung einzusetzen. Folgende Katalysatoren ausgewählt aus organischen Sn(IV)-, Sn(II)-, Zn-, Bi-Verbindungen oder tertiären Aminen können eingesetzt werden.

Bevorzugt werden Katalysatoren ausgewählt aus der Gruppe der Organozinnkatalysatoren, Titanate oder Zirkonate, metallorganische Verbindungen des Aluminiums, Eisens, Calciums, Magnesiums, Zinks oder Bismuths, Lewis-Säuren oder organische Säuren/Basen, lineare oder zyklische Amidine, Guanidine oder Amine oder eine Mischung daraus eingesetzt. Vorzugseise werden als Härtungs-Katalysatoren organische Zinnverbindungen, wie z.B. Dibutylzinndilaurat, Dibutylzinndiacetylacetonat, Dibutylzinndiacetat, Dibutylzinndioctoat, oder Dioctylzinndilaurat, Dioctylzinndiacetylacetonat, Dioctylzinndiketanoat, Dioctylstannoxan, Dioctylzinndicarboxylat, Dioctylzinnoxid, bevorzugt Dioctylzinndiacetylacetonat, Dioctylzinndilaurat, Dioctylzinndiketanoat, Dioctylstannoxan, Dioctylzinndicarboxylat, Dioctylzinnoxid, besonders bevorzugt Dioctylzinndiacetylacetonat und Dioctylzinndilaurat eingesetzt. Des Weiteren können auch Zinksalze, wie Zinkoctoat, Zinkacetylacetonat und Zink-2-ethylcaproat, oder Tetraalkylammoniumverbindungen, wie N,N,N-Trimethyl-N-2-hydroxypropyl-ammonium-hydroxid, N,N,N-Trimethyl-N-2-hydroxypropylammonium-2-ethylhexanoat oder Cholin-2-ethylhexanoat verwendet werden. Bevorzugt ist die Verwendung von Zinkoctoat (Zink-2-ethylhexanoat) und der Tetraalkylammoniumverbindungen, besonders bevorzugt diejenige von Zinkoctoat. Weiter bevorzugt sind Bismutkatalysatoren, z.B. TIB Kat (TIB Mannheim) oder Borchi®-Katalysatoren, Titanate, z.B. Titan(IV)isopropylat, Eisen(III)-Verbindungen, z.B. Eisen(III)-acetylacetonat, Aluminiumverbindungen, wie Aluminiumtriisopropylat, Aluminiumtri-secbutylat und andere Alkoholate sowie Aluminiumacetylacetonat, Calciumverbindungen, wie Calciumdinatriumethylendiamin-tetraacetat oder Calciumdiacetylacetonat, oder auch Amine, z.B. Triethylamin, Tributylamin, 1,4-Diazabicyclo[2,2,2]octan, 1,8-Diazabicyclo[5.4.0]undec-7-en, 1,5-Diazabicyclo[4.3.0]non-5-en, N,N-Bis-(N,N-dimethyl-2-aminoethyl)-methylamin, N,N-Dimethylcyclohexylamin, N,N-Dimethylphenylamin, N-Ethylmorpholin etc.. Auch organische oder anorganische Brönstedsäuren wie Essigsäure, Trifluoressigsäure, Methansulfonsäure, p-Toluolsulfonsäure oder Benzoylchlorid, Salzsäure, Phoshorsäure deren Mono- und/oder Diester, wie z.B. Butylphosphat, (Iso-)Propylphosphat, Dibutylphosphat etc., sind als Katalysatoren bevorzugt. Weiter bevorzugt sind Guanidin-Gruppen tragende organische und Silicium-organische Verbindungen. Selbstverständlich können auch Kombinationen mehrerer Katalysatoren eingesetzt werden. Darüber hinaus können auch photolatente Basen als Katalysatoren verwendet werden, wie sie in der WO 2005/100482 beschrieben sind.

Der Härtungskatalysator wird vorzugsweise in Mengen von 0,01 bis 5,0 Gew.-%, bevorzugt 0,05 bis 4,0 Gew.-% und besonders bevorzugt 0,1 bis 3 Gew.-% bezogen auf die Gesamtgewicht der härtbaren Zusammensetzung eingesetzt.

Bei filmbildenden Polymeren, die durch physikalische Trocknung aushärten, ist die Zugabe von reaktiven Härtern nicht erforderlich.

Die erfindungsgemäße Zusammensetzung können vorzugsweise in 1K-Beschichtungssysteme oder 2K- Beschichtungssysteme, in Melamin Einbrenn-Systeme, Raum- oder Hochtemperatursysteme eingesetzt werden.

Vorzugsweise weisen aus der erfindungsgemäßen Zusammensetzung hergestellte Beschichtungen eine antimikrobielle Wirkung gegen Bakterien, Hefen, Schimmelpilze, Algen, Parasiten und Viren auf.

Bevorzugt weisen die erfindungsgemäßen hergestellten Beschichtungen eine antimikrobielle Wirkung gegen
- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Fakultativ pathogene Umweltorganismen, vorzugsweise gegen *Cryptosporidium parvum, Giardia lamblia,* Amöben (*Arcanthamoeba* spp., *Naegleria* spp.), *E. coli,* coliforme Bakterien, Fäkalstreptokokken, *Salmonella* spp., *Shigella* spp., *Leginonella* spec., *Pseudomonas aeruginosa, Mykobakteria* spp., enterale Viren (z.B. Polio- und Hepatitis-A Virus);
- Pathogene in Lebensmitteln, vorzugsweise gegen *Bacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen, auf.

Ein weiterer Gegenstand ist die Verwendung der erfindungsgemäßen Zusammensetzung zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

Vorzugsweise ist die Verwendung der erfindungsgemäßen Zusammensetzung zur Herstellung von Beschichtungen mit anti-mikrobieller Eigenschaft.

Eine Beschichtung mit antimikrobieller Wirkung oder antimikrobieller Eigenschaft bedeutet hierbei, dass die Beschichtung eine antimikrobielle Oberfläche aufweist, die das Wachstum und die Vermehrung von Mikroorganismen begrenzt oder verhindert.

Es wurde erstaunlicherweise auch festgestellt, dass die erfindungsgemäßen Beschichtungen eine chemische und mechanische Beständigkeit aufweisen. Die chemische und mechanische Beständigkeit ist besonders bedeutsam, da antimikrobielle Beschichtungen häufig in Bereichen eingesetzt werden, die eine regelmäßige Desinfektion und weitere Hygienemaßnahmen erfordern.

Auch zur Erfindung gehört ein Verfahren zur Bildung einer anti-mikrobiellen Beschichtung auf einem Substrat, umfassend das Aufbringen einer härtbaren filmbildenden Zusammensetzung auf das Substrat, umfassend:
a. mindestens ein filmbildendes Polymer, das funktionelle Gruppen enthält, welche mit einem isocyanathaltigen Härter reaktiv sind, ggf. durch einen Katalysator katalysiert,
b. mindestens einen Leuchtstoff gemäß der Formel (II) und
c. einen Härter, enthaltend isocyanatfunktionelle Gruppen.

Vorzugsweise handelt es sich bei dem Substrat um Metall, mineralische Substrate (wie etwa Beton, Naturstein oder Glas), zellulosehaltige Untergründe, Holz sowie deren Hybride, formstabile Kunststoffe und/oder Duromere.

Unter dem Begriff "formstabile Kunststoffe" werden folgende, jedoch nicht abschließende, Polymere verstanden: Acrylnitril-Butadien-Styrol (ABS), Polyamide (PA), Polylactat (PLA), Polymethylmethacrylat (PMMA), Polycarbonat (PC), Polyethylenterephthalat (PET), Polystyrol (PS), Polyetheretherketon PEEK), Polyvinylchlorid (PVC). Polypropylen (PP), Polyethylen (PE).

Bevorzugt kann auf das Substrat vor der Auftragung der härtbaren filmbildenden Zusammensetzung eine Grundierzusammensetzung aufgetragen werden.

Vorzugsweise wird die erfindungsgemäße härtbare Zusammensetzung zur Beschichtung von Substraten in Hygieneeinrichtungen, Krankenhäusern und in der Lebensmittelindustrie eingesetzt.

Es können alle Bereiche des öffentlichen Raums, wie z.B. Schule, Altenheim, Großküche oder Kindergarten in Frage kommen.

Eine weitere Erfindung ist der Gegenstand, der mindestens teilweise, vorzugsweise vollständig, mit der erfindungsgemäßen härtbaren Zusammensetzung beschichtet ist.

Es sei hier anzumerken, dass die Begrifflichkeiten "antimikrobieller Effekt", antimikrobielle Wirksamkeit", "antimikrobielle Wirkung" und "antimikrobielle Eigenschaft" als Synonyme verwendet werden.

Nachfolgend werden Beispiele aufgeführt, die dem Fachmann allein zur Erläuterung dieser Erfindung dienen und stellen keinerlei Beschränkung des beanspruchten Gegenstandes dar.

### Methoden

### • Transmissionsmessung

Die Transmissionsmessungen wurden mit einem "Specord 200 Plus" UV/VIS-Zweistrahlspektrometer der Firma Analytik Jena bestimmt. Mit einem Holmiumoxidfilter erfolgt eine interne Wellenlängenkalibrierung. Die Proben wurden mit monochromatisiertem Licht einer Deuterium- (UV-Bereich) oder einer Wolfram-Halogen-Lampe (sichtbarer Bereich) durchstrahlt. Die spektrale Bandbreite beträgt 1,4 nm. Das monochromatisierte Licht wird in einen Mess- und einen Referenzkanal aufgeteilt und ermöglicht das direkte Messen gegen eine Referenzprobe. Die durch die Probe transmittierende Strahlung wird von einer Photodiode detektiert und verarbeitet. Die Messungen erfolgten im Transmissionsmodus. Der Messbereich betrug 190 bis 1100 nm mit einer Schrittweite von 1 nm. Die Messgeschwindigkeit lag bei 10 nm/s, was einer Integrationszeit von 0,1 s entspricht.

### Geräte

Speedmixer, Firma Hauschild Engineering Modell FAC 150.1 FVZ
Dispermat, Firma Getzmann Gerätetyp CV2-SIP
Glanzmessgerät, Firma Zehntner Testing Instruments Gerätetyp ZGM 1130
Gitterschnitttester, DIN EN ISO 2409, MTV Messtechnik oHG Typ: Gitterschnittschablonen Set CCP
Erichsen Tiefungstest, Firma Erichsen Typ 202
MEK Doppelhub Test, Fa. Bruno Pellizzato Typ: Tester Veslic Type
Rotationsviskosimeter, Firma Anton Paar Gerät Viskotherm VT 2
Spektralphotometer (für die Farbortbestimmung Messung), Fa. X-Rite Geräte Typ SP 62 Laborwaage, Sartorius MSE 6202 S 100 DO
Hämozytometer (Zählkammer nach Thoma): Fa. Brandt
Schüttelwasserbad: GFL 1083, Fa. Byk Gardner
Specord 200 Plus" UV/VIS-Zweistrahlspektrometer, Firma Analytik Jena

### Nährmedien

Caso-Bouillon: Fa. Merck KGaA Millipore
CASO Nähragarplatten: Fa. Oxoid

### Desinfektionsmittel

Bacillol® AF: Fa. Hartmann

### Materialien

**Tabelle 1: Übersicht der Rohstoffe für die verwendeten Polymermatrices**

| **Handelsname** | **Chemische Zusammensetzung** | **Verwendung** | **Unternehmen** |
|---|---|---|---|
| Degalan® 64/12 | Lufttrocknendes lineares Polyacrylat | Filmbildendes Polymer | Fa. Evonik |
| Polyimide P84®NT | Polyimidharz | Filmbildendes Polymer | Fa. Evonik |
| Desmophen® NH 1420 | Polyasparticsharz | Filmbildendes Polymer | Fa. Covestro |
| Dynacoll® AC 3820 | Hydroxylgruppenhaltiges lineares Polyacrylat | Filmbildendes Polymer | Fa. Evonik |
| Setal® 1603 | Hydroxylgruppenhaltiger Polyester | Filmbildendes Polymer | Fa. Allnex |
| CAB™ 381-2 | Celluloseester | Filmbildendes Polymer | Fa. Eastman |
| Desmodur® N 3390 | Polyisocyanat | Härter | Fa. Covestro |
| Butylacetat | Butylacetat | Lösemittel | Fa. Sigma-Aldrich |
| TIB KAT® 218 | Dibutylzinndilaurat | Katalysator | TIB Chemicals AG |

**Tabelle 2: Übersicht der verwendeten Additive**

| **Handelsname** | **chemische Zusammensetzung** | **Verwendung** | **Unternehmen** |
|---|---|---|---|
| TEGO® Dispers 710 | Lösung eines basischen Urethancopolymers | Dispergiermittel | Fa. Evonik |
| TEGO® Dispers 628 | Lösung eines hochmolekularen Polymers | Dispergiermittel | Fa. Evonik |
| TEGO® Dispers 670 | Lösung eines hochmolekularen Polymers | Dispergiermittel | Fa. Evonik |
| TEGO® Dispers 650 | Modifiziertes Polyether mit pigmentaffinen Gruppen | Dispergiermittel | Fa. Evonik |
| TEGO® Dispers 652 | Konzentrat eines Fettsäure-Derivats | Dispergiermittel | Fa. Evonik |
| TEGO® Dispers 630 | Lösung eines hochmolekularen AcrylsäurePolymers mit Aminderivat | Dispergiermittel | Fa. Evonik |
| TEGO® Dispers 688 | hochmolekulares Polymer | Dispergiermittel | Fa. Evonik |
| TEGO® Dispers 689 | hochmolekulares Polymer | Dispergiermittel | Fa. Evonik |
| TEGO® Dispers 1010 | hochmolekulares Polymer | Dispergiermittel | Fa. Evonik |
| SPHERILEX® DP0111 | synthetisches amorphes Silika | Füllstoff | Fa. Evonik |
| SPHERILEX® DP0112 | synthetisches amorphes Silika | Füllstoff | Fa. Evonik |
| AEROSIL® R 972 | Pyrogene Kieselsäure | Füllstoff | Fa. Evonik |
| AEROSIL® 200 | Pyrogene Kieselsäure | Füllstoff | Fa. Evonik |
| BENTONE® SD- 2 | Bentonit, Schichtsilikat | Füllstoff | Fa. Elementis |
| BENTONE® SD- 3 | Hectorit, Schichtsilikat | Füllstoff | Fa. Elementis |
| BENTONE® 38 | Modifiziertes Hectorit, Schichtsilikat | Füllstoff | Fa. Elementis |
| SPHERILEX™ DP0115 | synthetisches amorphes Silika | Füllstoff | Fa. Evonik |

### Abbildungen

**Abbildung 1****:** Transmissionsspektren der Polymermatrices P1 - P6 und Quarzglas ohne Beschichtung als Referenz
**Abbildung 2****:** Aufbau des Agarplatten Tests.
   Die Leuchtstoffprobe ( 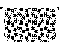 ) wird auf eine konfluent inokulierte Nähragarplatte ( 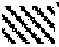 ) aufgetragen und unter konstanter Beleuchtung für 24 ± 1 h bei Raumtemperatur inkubiert. Zur Überprüfung der antimikrobiellen Wirksamkeit durch den Effekt der Up-conversion wurden die Proben zusätzlich im Dunkeln inkubiert.

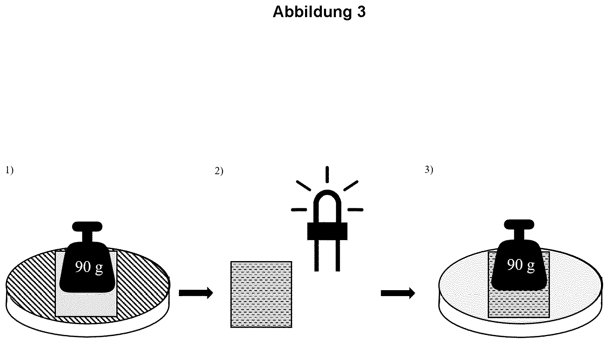

**Abbildung 3****:** Aufbau der Transfermethode.
   Die polymeren Matrices mit den enthaltenen Leuchtstoffen werden mit definiertem Gewicht auf eine konfluent inokulierte Nähragarplatte gedrückt (1). Die dadurch transferierten Bakterien werden unter Beleuchtung oder abgedunkelt bei Raumtemperatur inkubiert (2). Die Detektion des antimikrobiellen Effekts erfolgt über einen Abklatsch mit definiertem Gewicht auf Nähragar (3).

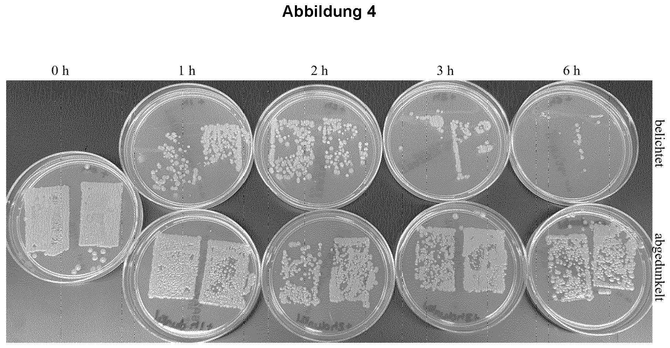

**Abbildung 4****:** Kultivierbarkeit von B. *subtilis* nach Inkubation auf der polymeren Matrix mit CaLi₂SiO₄:Pr³⁺,Na⁺(1%) im belichteten und abgedunkelten Zustand. *B. subtilis* wurde für 0h, 1h, 2h, 3h und 6h bei Raumtemperatur mit und ohne Belichtung inkubiert. Die anschließende Kultivierung der Zellen auf CASO-Agar fand für 24 ± 1 h bei 30 °C statt. Die Abbildung zeigt eine repräsentative Fotographie.

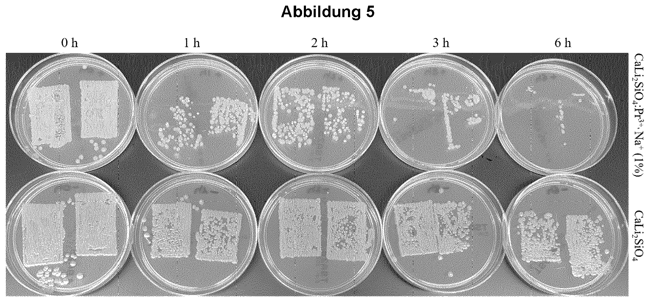

**Abbildung 5****:** Kultivierbarkeit von B. *subtilis* nach Inkubation auf der polymeren Matrix mit CaLi₂SiO₄:Pr³⁺,Na⁺(1%) und CaLi₂SiO₄ im belichteten Zustand. B. *subtilis* wurde für 0 h, 1 h, 2 h, 3 h und 6h bei Raumtemperatur mit ständiger Belichtung inkubiert. Die anschließende Kultivierung der Zellen auf CASO-Agar fand für 24 ± 1 h bei 30 °C statt. Die Abbildung zeigt eine repräsentative Fotographie.

### 1. Auswahl der filmbildenden Polymere

Anhand der gemessenen Transmission wurden die geeigneten filmbildende Polymere für die erfindungsgemäße Zusammensetzung ausgewählt.

### 1.1 Vorbereitung der Zusammensetzung ohne Leuchtstoffe und Additive

Es wurden Polymermatrices P1 - P6 wie folgt hergestellt, wobei es sich bei P1 und P2 um ein 1-Komponentensystem handelt, das physikalisch trocknet. Bei P3 - P6 handelt es sich um 2-Komponenten Systeme, die chemisch härtend sind.

Die Polymere aus Tabelle 1 wurden in den in Tabelle 3 aufgeführten Mengen in Butylacetat verdünnt bzw. gelöst. (Ausnahme: Polyimide P84®NT, das zu 100 % eingesetzt wurde). Anschließend wurden 20 g dieser Polymerlösung in 50 ml Kunststoffbecher eingewogen. Der Härter und/oder Katalysator wurde erst kurz vor der Applikation zugegeben. Die Polymermatrices wurden dann für 1 min bei 2000 rpm im Speedmixer homogenisiert.

**Tabelle 3: Zusammensetzung von jeweils 100 g der Polymermatrices P1 - P6**

| **Rohstoffe [g]** | **P1** | **P2** | **P3** | **P4** | **P5** | **P6** |
|---|---|---|---|---|---|---|
| Degalan® 64/12 | 73,2 | | | | | |
| Polyimide P84®NT | | 100 | | | | |
| Desmophen® NH 1420 | | | 44,2 | | | |
| Dynacoll® AC 3820 | | | | 32,2 | | |
| Setal® 1603 | | | | | 54,9 | |
| CAB™ 381-2 | | | | | | 27,2 |
| Desmodur® N 3390 | | | 33,6 | 4 | 31,2 | 9,1 |
| Butylacetat | 26,8 | | 22,1 | 63,70 | 13,8 | 63,6 |
| TIB KAT® 218 | | | 0,1 | 0,1 | 0,1 | 0,1 |

### 1.2 Beschichtung der Polymermatrices auf Quarzplatten

P1 - P6 wurden mit einem geeigneten Spiralrakel auf Quarzplatten aufgetragen, so dass im getrockneten Zustand eine Trockenschichtdicke von 30 µm erzielt wurde. Diese wurden bei Raumtemperatur (23°C) für 10 Tage getrocknet bzw. gehärtet.

### 1.3 Transmissionsmessung

Anschließend wurde das UV/VIS Transmissionsspektrum gemessen.

Die Polymermatrices P1, P4 und P6 zeigen eine hohe Transmission im Wellenlängenbereich 450 bis 500 nm (Blaulicht) und 250 bis 300 nm (UV-C/B Licht) **(Fehler! Verweisquelle konnte nicht gefunden werden.)** und Tabelle 4 zeigt die Transmissionen bei einer Wellenlänge bei 260 nm und 500 nm.

P1, P4 und P6 weisen eine Transmission bei beiden Wellenlängen von größer als 80% auf. Somit können die filmbildenden Polymere Degalan® 64/12 (in P1), Dynacoll® AC 3820 (in P4) und CAB™ 381-2P6 (in P6) für die erfindungsgemäße Zusammensetzung zur Herstellung von Beschichtungen mit antimikrobieller Eigenschaft eingesetzt werden. Polyimide P84®NT in P2 kann als Vergleichspolymer dienen, da dieses Polymer keine Transmission bei der Wellenlänge 260 nm aufweist.

**Tabelle 4 Übersicht der Transmission bei 260 nm und 500 nm**

| **Polymermatrix** | **Transmission 260 nm [%]** | **Transmission 500 nm [%]** |
|---|---|---|
| P1 | 89,45 | 93,3 |
| P2 | 0 | 89,2 |
| P3 | 57,72 | 92,4 |
| P4 | 84,85 | 92,2 |
| P5 | 54,45 | 92,4 |
| P6 | 86,31 | 92,8 |

### 2. Auswahl der Additive

Zur Optimierung der Lackeigenschaften und Stabilisierung der Leuchtstoffe, beispielsweise gegen Absetzen in der flüssigen erfindungsgemäßen Zusammensetzung, wurden verschiedene Additive in der Polymermatrix P4 überprüft. Neben der funktionellen Eignung der Additive wurde ihre Eignung hinsichtlich ihres Einflusses auf die Transmission überprüft. Hierfür wurden die UV/VIS Transmissionsspektren der Formulierungen verschiedener Additive in der Polymermatrix P4 gemessen.

### 2.1 Transmissionsmessung

Dazu wurden 20 g der Polymermatrix P4 und die Menge des zu überprüfenden Additivs eingewogen (siehe Tabelle 5) und im Speedmixer für 1 min bei 2000 rpm homogenisiert. Kurz vor der Applikation wurden Härter und Katalysator zugegeben und nochmals für 1 min bei 2000 rpm im Speedmixer homogenisiert. Diese Mischungen P4-1 bis P4-17 wurden auf Quarzglasplatten und Aluminium Bleche mit einem Spiralrakel appliziert und für 10 Tage bei Raumtemperatur getrocknet/gehärtet. Sie wurden hinsichtlich ihrer Transmission und Lackeigenschaften überprüft.

Anhand der UV/VIS Transmissionssprektren sind die Additive TEGO® Dispers 628, TEGO® Dispers 670, TEGO® Dispers 688, SPHERILEX® DP0111, SPHERILEX® DP0112, SPHERILEX® DP0115, AEROSlL® R 972, AEROSlL® 200, BENTONE SD®-2, BENTONE SD®-3und BENTONE® 38 für die erfindungsgemäße Zusammensetzung geeignet, da sie die erforderliche Transmission, auch im Hinblick auf die Transmission der filmbildenden Polymere (siehe Tabelle 2) nicht signifikant reduzieren. Deren Transmissionen sind größer als 70%.

Die Transmissionen, die kleiner als 70% sind, konnten bei den Additiven TEGO® Dispers 710, TEGO® Dispers 650, TEGO® Dispers 652, TEGO® Dispers 630, TEGO® Dispers 689 und TEGO® Dispers 1010 gemessen werden. (Tabelle 5).

**Tabelle 5: Übersicht der UV/VIS Transmission [%] der Zusammensetzung aus dem filmbildenden Polymer P4 mit Additiven bei 260 nm und 500 nm.**

| | **Additiv Produktname** | **Menge des Additiv [g]** | **Transmission 260 nm [%]** | **Transmission 500 nm [%]** |
|---|---|---|---|---|
| P4 | | ohne | 84,85 | 92,2 |
| P4-1 | TEGO® Dispers 710 | 2,86 | 0,4 | 84,41 |
| P4-2 | TEGO® Dispers 628 | 2 | 79,49 | 91,7 |
| P4-3 | TEGO® Dispers 670 | 2,5 | 72,39 | 92,72 |
| P4-4 | TEGO® Dispers 650 | 1 | 51,11 | 92,62 |
| P4-5 | TEGO® Dispers 652 | 1 | 35,52 | 92,3 |
| P4-6 | TEGO® Dispers 630 | 2 | 47,77 | 86,02 |
| P4-7 | TEGO® Dispers 688 | 2,2 | 79,49 | 92,62 |
| P4-8 | TEGO® Dispers 689 | 2,2 | 55,79 | 89,64 |
| P4-9 | TEGO® Dispers 1010 | 1 | 69,84 | 85,05 |
| P4-10 | SPHERILEX® DP0111 | 0,4 | 75,2 | 88,25 |
| P4-11 | SPHERILEX® DP0112 | 0,4 | 77,89 | 89,21 |
| P4-12 | AEROSIL® R 972 | 0,4 | 82,49 | 92,06 |
| P4-13 | AEROSIL® 200 | 0,4 | 84,28 | 92,51 |
| P4-14 | BENTONE SD®-2 | 0,4 | 76,72 | 91,24 |
| P4-15 | BENTONE SD®-3 | 0,4 | 74,59 | 91,11 |
| P4-16 | BENTONE® 38 | 0,4 | 77,93 | 91,22 |
| P4-17 | SPHERILEX® DP0115 | 0,4 | 77,18 | 89,04 |

### 2.2 Überprüfung der Lackeigenschaften der Polymermatrices ohne Leuchtstoffe

Die flüssigen Polymermatrices wurden mit einem Spiralrakel auf Bonder 26s 6800 OC Blechen appliziert und bei 23°C für 10 Tage getrocknet/gehärtet. Es wurde eine finale Trockenschichtdicke von 30 µm erzielt.

Folgende Lackeigenschaften wurden in Anlehnung an gängige DIN und ISO-Normen überprüft:
- Glanz
- Pendelhärte nach König
- Gitterschnitttest
- Erichsen Tiefungstest
- MEK Doppelhub Test
- Chemikalienbeständigkeit gegen Ketchup, Kaffee, Schwefelsäure (50% Lösung in Wasser), Natronlauge (10% Lösung in Wasser) und Sonnencreme. Die Sonnencreme wurde nach dem Auftragen auf die Lackoberfläche 1 h lang 60°C im Ofen ausgesetzt, alle anderen Chemikalien verblieben für 16 h bei Raumtemperatur, bevor sie entfernt wurden und danach die Schädigung der Lackoberfläche bewertet wurde.
- Bacillol Doppelhub Test: Bacillol® AF eignet sich zur Schnelldesinfektion alkoholbeständiger Oberflächen im Sprüh-/Wischverfahren.

Die Lackeigenschaften wurden in den Polymermatrices P3 - P6 überprüft **(Fehler! Verweisquelle konnte nicht gefunden werden.).** Es wurde festgestellt, dass die Polymermatrices P4 und P6 den üblichen Lackeigenschaften genügen. Diese können daher für die weiteren Prüfungen eingesetzt werden.

**Tabelle 6: Lackeigenschaften von P3 bis P6**

| **Test** | **Norm** | **Beurteilungsgrundlage** | **Toleranz/übliche Werte** | **P4** | **P5** | **P3** | **P6** |
|---|---|---|---|---|---|---|---|
| Pendelhärte nach König | DIN EN ISO 1522 | Ausschläge | Weich: <90 Hart: >140 | 112 | 157 | 141 | 127 |
| Glanz 20° | DINEN ISO 2813 | Einheiten | Hochglanz: bei 20° >70E Semi-Glanz: bei 60° 10-70E | 88 | 86,8 | 91,1 | 97 |
| Gitterschnitt | DIN EN ISO 2409 | Visuell: Skala von 0 bis 5 | 0=ohne Abplatzen 5=völlig abgeplatzt | 0 | 5 | 5 | 0 |
| Tiefung nach Erichsen | DIN EN ISO 1520 | mm | Hart: <2mm Weich: >6mm | 2 | 2 | 4,8 | 2 |
| MEK Test | ASTM D 4752 | Doppelhübe | Niedrige Vernetzungsdichte: <50 Hohe Vernetzungsdichte: >200 | 113 | >200 | >200 | >200 |
| Bacillol-Test | Anlehnend an ASTM D 4752 | Doppelhübe | Niedrige Vernetzungsdichte: <50 Hohe Vernetzungsdichte: >200 | >200 | >200 | >200 | >200 |
| Beständigkeit gegen Ketchup | DIN EN ISO 175 | Visuell: Skala von 1 bis 10 | 1 =starke Veränderung 10=keine Veränderung | 10 | 10 | 10 | 10 |
| Beständigkeit gegen Kaffee | DIN EN ISO 175 | Visuell: Skala von 1 bis 10 | 1 =starke Veränderung 10=keine Veränderung | 10 | 10 | 10 | 10 |
| Beständigkeit gegen H₂SO₄ (50% in Wasser) | DIN EN ISO 175 | Visuell: Skala von 1 bis 10 | 1 =starke Veränderung 10=keine Veränderung | 10 | 10 | 10 | 10 |
| Beständigkeit gegen NaOH (10% in Wasser) | DIN EN ISO 175 | Visuell: Skala von 1 bis 10 | 1 =starke Veränderung 10=keine Veränderung | 10 | 10 | 10 | 10 |

### 3. Überprüfung der antimikrobiellen Wirksamkeit

### 3.1 Auswahl der Leuchtstoffe

Es wurden folgende Leuchtstoffe herangezogen:
- Lu₂CaAl₄SiO₁₂:Pr³⁺,Gd³⁺, hergestellt gemäß unveröffentlichter europäischen Patentanmeldung EP 19202897.5, Beispiel 6
- CaLi₂SiO₄:Pr³⁺,Na⁺(1%), hergestellt gemäß unveröffentlichter europäischen Patentanmeldung EP 19202910.6, Beispiel 1
- CaLi₂SiO₄, hergestellt gemäß unveröffentlichter europäischen Patentanmeldung EP 19202910.6, Beispiel 1, wobei kein Praseodym verwendet wurde.
- Li₄P₂O₇, hergestellt gemäß folgender Vorschrift:
   1,8473 g (25,0000 mmol) Li₂O₃ und 2,8756 g (25,000 mmol) NH₄H₂PO₄ wurden in Aceton in einer Achatreibschale vermischt. Diese vorbereitete Mischung wurde bei 500°C für 6h unter normaler (Luft)Atmosphäre kalziniert. Es wurde für weitere 12h bei 650°C unter normaler (Luft)Atmosphäre zur Erhaltung des Produktes kalziniert.
- BaY₂Sl₃O₁₀:Pr³⁺, hergestellt gemäß folgender Vorschrift:
   2,1273 g (10,7800 mmol) BaCO₃, 1,9828 g (33,0000 mmol) SiO₂, 2,4839 g (11,0000 mmol) and 0,0187 g (0,0183 mmol) Pr₆O₁₁ wurden in Aceton in einer Achatreibschale vermischt. Diese vorbereitete Mischung wurde bei 1400°C für 6h unter CO-Atmosphäre kalziniert zur Erhaltung des Produktes.
- Ca₃Sc₂Si₃0₁₂:Pr³⁺,Na⁺(1%), hergestellt gemäß folgende Vorschrift:
   1,8119 g (18,1030 mmol) CaCO₃, 0,0104 g (0,0102 mmol) Pr₆O₁₁, 0,8428 g (6,1110 mmol) S_{C2}O₃ and 0,0032 g (0,0306 mmol) Na₂CO₃ wurden in heiße konzentrierte Salpetersäure gelöst. Die Lösung wurde eingedampft, um die Nitrate zu erhalten. Unter ständigem Rühren wurde Wasser zu den Nitraten hinzugefügt. 1,1043 g (18,3790 mmol) SiO₂ wurde mit 20 mL Wasser vermischt und in einem Ultraschallbad zur Trennung der Agglomerate gesetzt. Diese Dispersion wurde zu der o. g. Wasser-NitratLösung zugeführt und vermischt. 11,1314 g (121,1300 mmol) C₄H₁₁NO₃ wurde dazu gegeben. Die Lösung wurde eingedampft. Das Reaktionsprodukt wurde bei 150°C getrocknet. Es wurde dann bei 1000°C in einem Muffelofen für 2h unter normaler (Luft)Atmosphäre kalziniert. Ein weiterer Kalzinierungsschritt wurde bei 1300 °C für 4h unter einem Formiergas (N₂/H_{2;} 95% /5%) zur Erhaltung des Produktes durchgeführt.
- Es wurde der Leuchtstoff entsprechend der WO 2009/064845 A2 hergestellt:
   3.3349 g (33.3200 mmol) CaCO₃, 2.5123 g (34.0000 mmol) Li₂CO₃, 0.1479 g (0.3400 mmol) Pr(NO₃)₃·6H₂O und 0.0180 g (0.1700 mmol) Na₂CO₃ werden in Hexan in einer Achatreibschale vermischt. Na₂CO₃ wurde zur Ladungkompensation von Ca^{2+/}Pr³⁺ zugesetzt. Diese Mischung wurde bei 700°C für zwei Stunden bei Luftzufuhr zur Entfernung organische Bestandteile kalziniert. Anschließend wird die Kalzinierung bei 1100°C (und höher) für 12h durchgeführt, was zu einem amorphen und glasartigen Produkt geführt hat, das im Tiegel festklebte. Es war nicht möglich, das amorphe Produkt vom Al₂O₃ Tiegel zu trennen.

Somit ist der Leuchtstoff gemäß der WO 2009/064845 A2 für die erfindungsgemäße Zusammensetzung nicht geeignet. Diese konnte nicht für die weiteren Untersuchungen eingesetzt werden.

### 3.2 Überprüfung der antimikrobiellen Wirksamkeit der Leuchtstoffe

Zunächst wurde die antimikrobielle Wirksamkeit der Leuchtstoffe als solche getestet. Es wurde die Wirksamkeit der Leuchtstoffe gegen gram-positive und gram-negative Testorganismen getestet.

Geprüft wurde an *Bacillus subtilits,* der im DVGW (Deutscher Verein des Gas- und Wasserfaches), Arbeitsblatt W 294 "UV-Geräte zur Desinfektion in der Wasserversorgung" zur biodosimetrischen Prüfung von UV-Anlagen eingesetzt wird. Als gram-positives sporenbildendes Bakterium ist er besonders unempfindlich gegenüber UV-Strahlung und damit als "worst case" zur Überprüfung der antimikrobiellen Wirkung von UV-Strahlung gut geeignet.

Des Weiteren wurde die antimikrobielle Wirksamkeit an *Escherichia coli* getestet, um die antimikrobielle Wirkung gegen gram-negative Bakterien darzustellen. *E. coli* ist ein gramnegatives aerobes Bakterium, welches prädominant im menschlichen Intestinaltrakt vorkommt und ist damit ein typischer Fäkalkontaminationsindikator. Bei Kontamination von anderen Geweben mit *E. coli* kommt es häufig zu Infektionskrankheiten, wie zum Beispiel Infektionen im Urogenitaltrakt.

### 3.2.1 Agarplatten Test

Anhand des Agarplatten Test wurde die antimikrobielle Wirkung der Leuchtstoffe auf die Testorganismen *B. subtilis* und *E. coli* überprüft.

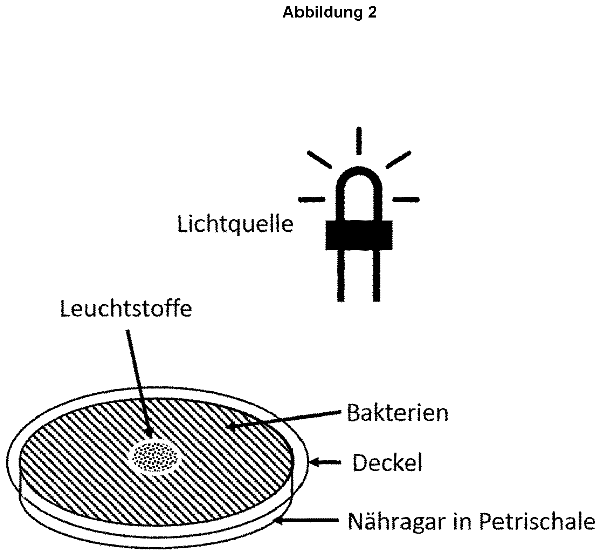

Zur Testung wurden feste Nähragarplatten mit einer Bakteriensuspension der Testorganismen konfluent inokuliert. Auf die inokulierten Nährböden wurden die Leuchtstoffproben gegeben (Abbildung 2). Die Platten wurden bei geeigneten Wachstumsbedingungen inkubiert. Nach der Inkubation der Platten wurden die wachstumsinhibierenden Eigenschaften anhand der Bildung einer Zone ohne Koloniewachstum konzentrisch auf und um die angehäuften Leuchtstoffe auf den Nährböden bewertet.

Als Testorganismen wurden *Bacillus subtilis* subsp. spizizenii (DSM 347, ATCC 6633) und *Escherichia coli* (DSM 1116; ATCC 9637) verwendet. Die Testorganismen wurden in Suspension mit einer finalen Konzentration von 10⁷ Zellen/ml verwendet.

Die Bakteriensuspensionen wurden durch Verdünnungen von Vorkulturen des jeweiligen Bakterienstammes hergestellt. Verdünnt wurde in sterilem deionisierten Wasser. Die Vorkulturen der Testorganismen wurden in sterilisierter Caseinpepton-Sojamehlpepton (CASO) Bouillon hergestellt. Die Vorkultur von *B. subtilis* wurde für 16 ± 1 h bei 30 °C unter konstantem Schütteln im Schüttelwasserbad inkubiert. Die Vorkultur von *E. coli* wurde bei 36 °C in einem thermisch isolierten Erlenmeyerkolben mit Magnetrührfisch unter konstantem Rühren bei 350 rpm inkubiert. Der Zelltiter der Vorkulturen wurde mikroskopisch mit einem Hämozytometer (Zählkammer nach Thoma) bestimmt.

Für den Agarplattentest wurden 1.0 ml der Bakteriensuspension mit 10⁷ Zellen/ml auf einer sterilen CASO Agarplatte gleichmäßig verteilt, um eine konfluente Belegung des Nähragars zu gewährleisten. Die aufgetragene Bakteriensuspension wurde bei 300 ± 30 sec bei Raumtemperatur (22 ± 2 °C) auf dem Nähragar equilibriert, bevor die Leuchtstoffe zentriert aufgetragen wurden. Zudem wurden jeweils Calciumcarbonat und Kupferoxid als negativ- und positiv-Referenz zentriert auch die Nährplatten aufgetragen. Es ist bekannt, dass Kupferoxide einen wachstumsinhibierenden Effekt aufweisen, während Calciumcarbonate keinen wachstumsinhibierenden Effekt aufzeigen dürfen.

Die Nährplatten wurden für 24 ± 1 h bei Raumtemperatur unter konstanter Beleuchtung inkubiert. Der gleiche Ansatz wurde zusätzlich auch im Dunkeln inkubiert.

Das Inkubieren unter Beleuchtung und im Dunkel soll, sofern ein wachstumsinhibierender Effekt nur im belichteten Zustand vorhanden ist, auf die up-conversion Eigenschaft der Leuchtstoffe hinweisen.

Alle Proben und Referenzen wurden im Triplikat sowie mit und ohne Beleuchtung während der Inkubationszeit von 24 ±1 h geprüft.

Leuchtstoffe und Leuchtstoffpartikel werden als Synonyme verwendet.

### 3.2.2 Ergebnisse des Agarplatten Tests

Der wachstumsinhibierende Effekt der Leuchtstoffe auf Bakterien wurde nach 24 ± 1 h bei Raumtemperatur visuell detektiert (Tabelle 7).

Ein wachstumsinhibierender Effekt ist dann gegeben, wenn eine Zone ohne bakterielles Koloniewachstums konzentrisch um und auf dem angehäuften Leuchtstoffpartikel bzw. Referenzpartikel auf dem Nähragar entsteht.

Kein wachstumsinhibierender Effekt ist dann gegeben, wenn um und auf den angehäuften Leuchtstoffpartikeln bzw. Referenzpartikeln bakterielles Koloniewachstum auf dem Nähragar detektiert wird.

Nach der Inkubation unter Beleuchtung nach 24 ± 1 h bei Raumtemperatur konnte ein wachstumsinhibierender Effekt des Leuchtstoffes CaLi₂SiO₄:Pr³⁺,Na⁺(1%) für *B. subtilis* und *E. coli* detektiert werden. Um die anderen Leuchtstoffe konnte keine wachstumsinhibierende Wirkung detektiert werden (Tabelle 7).

Für alle Leuchtstoffe konnte um und auf den angehäuften Leuchtstoffpartikeln kein bakterielles Koloniewachstum unter abgedunkelten Inkubationsbedingungen detektiert werden.

Diese Ergebnisse zeigen eindeutig, dass die antimikrobielle Wirkung der Leuchtstoffe CaLi₂SiO₄:Pr³⁺,Na⁺(1%) durch den physikalischen Effekt der UV-Emission im lichtangeregten Zustand begründet ist. Im abgedunkelten Zustand findet keine Up-conversion statt, sodass keine antimikrobielle Wirkung der Leuchtstoffe im abgedunkelten Zustand detektiert werden konnte.

Es ist des Weiteren festzuhalten, dass der Leuchtstoff CaLi₂SiO₄ keine wachstumsinhibierende Wirkung auf die Testorganismen zeigte. Demnach kann die Schlussfolgerung gezogen werden, jedoch ohne an einer Theorie gebunden zu sein, dass die Dotierung der Leuchtstoffe mit Praseodym vorteilhaft für die physikalische Effektivität der Up-conversion der Leuchtstoffe ist.

Die Referenz mit Calciumcarbonat zeigte weder im Hellen noch im Dunkeln eine Zone mit bakterieller Wachstumsinhibierung. Dagegen zeigt die Referenz mit Kupferoxid eine konzentrische Zone ohne bakterielles Koloniewachstum sowohl im Hellen als auch im Dunkeln.

Die Leuchtstoffe zeigten zu dem keine genuine bakterielle Kontamination.

Die Ergebnisse zeigen, dass für die erfindungsgemäße härtbare Zusammensetzung der Leuchtstoff CaLi₂SiO₄: Pr³⁺, Na⁺(1%) geeignet ist.

**Tabelle 7: Ergebnisse des Agarplatten Tests**

| **Leuchtstoff** | **Wachstumsinhibierende Wirkung auf *B. subtilis*** | | **Wachstumsinhibierende Wirkung auf *E*. *coli*** | |
|---|---|---|---|---|
| | **Beleuchtet** | **Abgedunkelt** | **Beleuchtet** | **Abgedunkelt** |
| Lu₂CaAl₄SiO₁₂:Pr³⁺,Gd³⁺ | Nein | Nein | Nein | Nein |
| CaLi₂SiO₄:Pr³⁺, Na⁺(1%) | Ja | Nein | Ja | Nein |
| CaLi₂SiO₄ | Nein | Nein | Nein | Nein |
| Li₄P₂O₇ | Nein | Nein | Nein | Nein |
| BaY₂Sl₃O₁₀:Pr³⁺ | Nein | Nein | Nein | Nein |
| Ca₃Sc₂Si₃O₁₂: Pr³⁺, Na⁺ | Nein | Nein | Nein | Nein |
| Referenz Calciumcarbonat | Nein | Nein | Nein | Nein |
| Referenz Kupferoxid | Ja | Ja | Ja | Ja |

### 3.3 Überprüfung der antimikrobiellen Wirksamkeit einer erfindungsgemäßen Zusammensetzung

Es konnte unter 3.2 gezeigt werden, dass der Leuchtstoff CaLi₂SiO₄:Pr³⁺, Na⁺(1%) als solche einen antimikrobiellen Effekt besitzt. Ob dieser antimikrobiellen Effekt jedoch auch in der erfindungsgemäßen Zusammensetzung weiterhin besteht, soll dies nun ermittelt werden.

Es sei hier anzumerken, dass die Begrifflichkeiten "antimikrobieller Effekt", antimikrobielle Wirksamkeit", "antimikrobielle Wirkung" und "antimikrobielle Eigenschaft" als Synonyme verwendet werden.

Zur Überprüfung der antimikrobiellen Wirksamkeit der erfindungsgemäßen Zusammensetzung, werden drei Leuchtstoffe und die filmbildenden Polymermatrices P4, P2 und P6 verwendet, wobei P2 als Vergleichsbeispiel dient.

Es wurden die Leuchtstoffe CaLi₂SiO₄:Pr³⁺,Na⁺(1%), Lu₂CaAl₄SiO₁₂:Pr³⁺,Gd³⁺ und CaLi₂SiO₄ eingesetzt, wobei lediglich CaLi₂SiO₄:Pr³⁺,Na⁺(1%) als erfindungsgemäßer Leuchtstoff fungiert.

### 3.3.1 Herstellung einer härtbaren Zusammensetzung

Die erfindungsgemäßen härtbaren Zusammensetzungen Z4-2 und Z6-2 sowie die Vergleichsbeispiele VZ4-1, VZ4-3, VZ2-1, VZ2-2, VZ2-3, VZ6-1 und VZ6-3 wurden gemäß den Angaben aus Tabelle 8 hergestellt. 50 g Glasperlen wurden zu der jeweiligen Zusammensetzung gegeben und für 5 min bei 2000 rpm im Speedmixer angerieben. Nach Abfiltrieren der Glasperlen wurde die jeweilige Zusammensetzung auf eine Kunststofffolie aufgezogen und zu einem Film vernetzt. Auf dem Substrat ist nun eine Beschichtung, deren Beschichtungsoberfläche eine antimikrobielle Wirkung aufweisen soll.

Die Rezeptur der Zusammensetzungen ist aus Tabelle 8 ersichtlich.

### 3.3.2 Transfermethode

Als Testorganismus wurde wiederum *Bacillus subtilis* subsp. spizizenii (DSM 347, ATCC 6633) verwendet. Es wurde 1 ml einer *B. subtilis* Suspension mit einer finalen Konzentration von 10⁷ Zellen/mL auf einer sterilen CASO Agarplatte gleichmäßig verteilt, um eine konfluente Belegung des Nähragars zu gewährleisten. Die aufgetragene Bakteriensuspension wurde bei 300 ± 30 sec bei Raumtemperatur (22 ± 2 °C) auf dem Nähragar equilibriert. Die Bakteriensuspension wurden durch Verdünnungen von Vorkulturen des jeweiligen Bakterienstammes hergestellt. Verdünnt wurde in sterilem deionisierten Wasser. Die Vorkulturen der Testorganismen wurden in sterilisierter CASO Bouillon hergestellt. Die Vorkultur von *B. subtilis* wurde für 16 ± 1 h bei 30 °C unter konstantem Schütteln im Schüttelwasserbad inkubiert. Der Zelltiter der Vorkulturen wurde mikroskopisch mit einem Hämozytometer (Zählkammer nach Thoma) bestimmt.

Das Ziel der Transfermethode ist es, die antimikrobielle Wirkung der Beschichtungsoberfläche unter realitätsnahen Bedingungen auf einer trockenen unbelebten Oberfläche zu simulieren. Hierfür wurden die, wie oben beschrieben, erhaltenen Beschichtungen auf eine Größe von 2,5 cm x 4 cm zugeschnitten und auf eine konfluent mit *B. subtilis* inokulierte Nähragarplatte mit einem definierten Gewicht von 90 ± 1 g für 60 ± 5 sec gedrückt. Durch diesen Schritt wurden die Bakterien semi-trocken auf die Oberfläche der Beschichtung übertragen. Anschließend wurden die Substrate mit der beschichteten und inokulierten Seite nach oben in eine leere Petrischale gelegt und unter Beleuchtung bei Raumtemperatur für 0h, 1h, 2h, 3h, 6h inkubiert.

Zur Überprüfung der antimikrobiellen Wirkung durch den Effekt der Up-conversion wurden die Substrate mit der beschichteten und inokulierten Seite zusätzlich auch im Dunkeln bei Raumtemperatur für 0h, 1h, 2h, 3h, 6h inkubiert.

Als Kontrollreferenzen wurden wiederum Calciumcarbonat (ohne wachstumsinhibierende Wirkung) und Kupferoxid (mit wachstumsinhibierender Wirkung) ausgewählt

Alle Proben und Referenzen wurden im Triplikat sowie mit und ohne Beleuchtung während der Inkubationszeit geprüft.

Die Detektion des antimikrobiellen Effekts nach der entsprechenden Inkubationszeit erfolgt über die Bestimmung der Kultivierbarkeit mit einen Abklatsch Test (Abbildung 3).

Für die Überprüfung der Kultivierbarkeit von *B. subtilis* wurden die Substrate mit der beschichteten und inokulierten Seite nach der Inkubationszeit von 0, 1, 2, 3, 6 h für 60 ± 5 sec mit einem definierten Gewicht von 90 ± 1 g auf eine sterile Nähragarplatte gedrückt. Der Nähragar wurde anschließend für 24 ± 1 h bei 30 °C statisch inkubiert. Die entstandenen bakteriellen Kolonien wurden visuell qualitativ ausgewertet.

**Tabelle 8: Rezepturen der härtbaren Zusammensetzungen für die Transfermethode**

| | **VZ4-1 [g]** | **Z4-2 [g]** | **VZ4-3 [g]** | **VZ2-1 [g]** | **VZ2-2 [g]** | **VZ2-3 [g]** | **VZ6-1 [g]** | **Z6-2 [g]** | **VZ6-3 [g]** |
|---|---|---|---|---|---|---|---|---|---|
| Dynacoll® AC 3820 | 16,00 | 16,00 | 16,00 | | | | | | |
| Polyimide P84®NT | | | | 50,00 | 50,00 | 50,00 | | | |
| CAB™ 381-2 | | | | | | | 6,82 | 6,82 | 6,82 |
| Butylacetat | 32,00 | 32,00 | 32,00 | | | | 38,64 | 38,64 | 38,64 |
| Lu₂CaAl₄SiO₁₂:Pr³⁺,Gd³⁺ | 1,33 | | | 1,33 | | | 0,90 | | |
| CaLi₂SiO₄:Pr³⁺,Na⁺(1 %) | | 1,33 | | | 1,33 | | | 0,90 | |
| CaLi₂SiO₄ | | | 1,33 | | | 1,33 | | | 0,90 |
| TIB Kat® 218 | 0,03 | 0,03 | 0,03 | | | | 0,05 | 0,05 | 0,05 |
| Desmodur® N 3390 | 2,00 | 2,00 | 2,00 | | | | 4,55 | 4,55 | 4,55 |

### 3.3.3 Ergebnisse der Transfermethode

Ein wachstumsinhibierender Effekt kann bei der Transfermethode durch eine Abnahme der Kultivierbarkeit von *B. subtilis* überprüft werden.

Die Kultivierbarkeit der adhärenten Bakterien auf der Beschichtungsoberfläche von Z4-2 und Z6-2 zeigten eine deutliche Vermehrungsreduktion mit steigender Inkubationsdauer (Abbildung 4). Der Leuchtstoff CaLi₂SiO₄:Pr³⁺,Na⁺(1%) bewirkt in der erfindungsgemäßen härtbaren Zusammensetzung eine signifikante Verminderung der Kultivierbarkeit von *B. subtilis* im Vergleich zur Nullprobe und den im Dunkeln inkubierten Proben. Diese Verminderung konnte bereits nach 1 h Inkubation unter ständiger Beleuchtung gemessen werden. Die Abnahme der Kultivierbarkeit erhöht sich bis zu der Inkubationszeit von 6 h bei ständiger Beleuchtung. Die im Dunkeln inkubierten Zusammensetzungen zeigten über den Inkubationszeitraum von 6 h keine Verminderung der Kultivierbarkeit. Repräsentative Bilder sind in Abbildung 4 für Z4-2 dargestellt.

Durch die unveränderte Anzahl der kultivierbaren Bakterien über den Zeitraum von 6 h kann gezeigt werden, dass der antimikrobielle Effekt des Leuchtstoffs nur im belichteten Zustand vorliegt. Der Up-conversion Effekt ist somit auch hier gegeben.

Die Vergleichs-Leuchtstoffe Lu₂CaAl₄SiO₁₂:Pr³⁺,Gd³⁺ und CaLi₂SiO₄ zeigten in keiner der Vergleichszusammensetzungen eine antimikrobielle Wirksamkeit, weder im belichteten noch im abgedunkelten Zustand (Tabelle 9).

Bei der Vergleichszusammensetzung VZ2-2 konnte keine antimikrobielle Wirkung für die getesteten Leuchtstoffe detektiert werden (Tabelle 9). Daraus ableitend kann festgehalten werden, dass das Polymer Polyimide P84®NT im Gegensatz zu dem Polymer Dynacoll® AC 3820 und dem Polymer CAB™ 381-2 kein geeignetes filmbildendes Polymer für die erfindungsgemäßen härtbaren Zusammensetzung darstellt.

Bei der Referenz mit Calciumcarbonat konnte keine Reduzierung der Kultivierbarkeit von *B. subtilis* im belichteten oder abgedunkelten Zustand detektiert werden. Durch Kupferoxid Zugabe konnte eine deutliche Reduzierung der Kultivierbarkeit sowohl im angedunkeltem als auch im belichteten Zustand detektiert werden.

Die polymeren Matrices zeigten zu dem keine genuine Kontamination.

Abbildung 5 zeigt auch hier, dass der Leuchtstoff CaLi₂SiO₄ keine antimikrobielle Wirkung aufweist.

**Tabelle 9: Antimikrobielle Wirksamkeit der härtbaren Zusammensetzungen**

| **Zusammensetzung** | **Antimikrobieller Effekt** | |
|---|---|---|
| | **Beleuchtet** | **Abgedunkelt** |
| VZ4-1 | Nein | Nein |
| Z4-2 | Ja | Nein |
| VZ4-3 | Nein | Nein |
| VZ2-1 | Nein | Nein |
| VZ2-2 | Nein | Nein |
| VZ2-3 | Nein | Nein |
| VZ6-1 | Nein | Nein |
| Z6-2 | Ja | Nein |
| VZ6-3 | Nein | Nein |
| Referenz Calciumcarbonat | Nein | Nein |
| Referenz Kupferoxid | Ja | Ja |

### 4. Physikalische Eigenschaften der erfindungsgemäßen Zusammensetzung

Eine wichtige Eigenschaft von härtbaren Zusammensetzungen ist die Lagerstabilität. Eine Aussage über die Lagerstabilität kann durch Messung der Viskosität und der Charakterisierung des Bodensatzes, wie Homogenisierung und Bildung eines Serums der erfindungsgemäßen härtbaren Zusammensetzung Z4-2 gemäß Tabelle 8, wobei kein Härter und kein Katalysator verwendet wurden, getroffen werden. Im Weiteren Z4-2* bezeichnet. Es wurde der Leuchtstoff CaLi₂SiO₄:Pr³⁺,Na⁺(1%) eingesetzt.

### Viskosität

Die Viskosität der Z4-2*, ohne Härter und Katalysator, mit den jeweiligen Additiven wurden mittels Rotationsviskosimeter Kegel-Platte gemessen. Die Differenz der Viskosität zum anfänglichen Wert, direkt nach dem Mischen, wurde nach einer Standzeit von 1 Woche und 2 Wochen bei 40 °C überprüft (Tabelle 10).

Es konnte festgestellt werden, dass alle Zusammensetzungen mit Additiven eine verbesserte Stabilität hinsichtlich der Viskosität bei 40°C Lagerung gegenüber der Zusammensetzung ohne Additiv zeigten. Die größte Stabilität hinsichtlich der Viskosität bei 40°C Lagerung zeigte die Zusammensetzung Z4-2* und Tego® Dispers 688. Die anfängliche Viskosität reduziert sich sogar leicht und garantiert eine gute Lagerstabilität.

### Sedimentation und Homogenisierung

Ergänzend wurde die Bildung eines Sedimentes nach einer Standzeit von 1 Woche und 2 Wochen bei 40 °C überprüft (Tabelle 11).

Auswertungskriterien:
Sediment [%] = Höhe [cm] des Sediments in Verhältnis zur Gesamthöhe [cm] des Nasslacks
Homogenisierung = leicht oder schwer, hierbei wurde mit einem Spatel gerührt.

Wie man aus Tabelle 11 erkennen kann, zeigten die Zusammensetzung Z4-2* und Tego® Dispers 688 und Z4-2* und Tego® Dispers 670 hinsichtlich der Sedimentation der Partikel sehr gute Ergebnisse. Es findet innerhalb von 2 Wochen bei 40°C keine messbare Sedimentation der Partikel statt. Bei Z4-2* und Tego® Dispers 628 konnten die Partikel nach 1 Woche Standzeit wieder leicht homogenisiert werden, was bei der Zusammensetzung ohne Additiv nicht der Fall war.

**Tabelle 10: Viskositäten [mPa]**

| **Additiv** | **Additivgehalt [% Festkörper]** | **Viskosität [mPa] nach Mischen (t = 0 Woche)** | **Δ Viskosität [mPa] t= 1 Woche bei 40°C** | **Δ Viskosität [mPa] t= 2 Wochen bei 40°C** |
|---|---|---|---|---|
| Z4-2* | 0 | 9489 | +1512,9 | +5803,9 |
| Z4-2* und Tego® Dispers 628 | 5 | 237 | +69,79 | +144,92 |
| Z4-2* und Tego® Dispers 670 | 5 | 3605 | +248,9 | +552,7 |
| Z4-2* und Tego® Dispers 688 | 5 | 4517 | -191,6 | -201,8 |

**Tabelle 11: Sedimentation und Homogenisierung**

| **Additiv** | **Additivgehalt [% Festkörper]** | **Sedimentation [%] t= 1 Woche bei 40°C** | **Homogenisierung t= 1 Woche bei 40°C** | **Sedimentation [%] t= 2 Wochen bei 40°C** | **Homogenisierung t= 2 Wochen** |
|---|---|---|---|---|---|
| Z4-2* | 0 | 5 | schwer | 5 | schwer |
| Z4-2* und Tego® Dispers 628 | 5 | 5 | leicht | 5 | schwer |
| Z4-2* und Tego® Dispers 670 | 5 | Keine Sedimentation | | Keine Sedimentation | |
| Z4-2* und Tego® Dispers 688 | 5 | Keine Sedimentation | | Keine Sedimentation | |

## Patentansprüche

1. Härtbare Zusammensetzung zur Herstellung von Beschichtungen mit anti-mikrobieller Eigenschaft enthaltend
- mindestens ein filmbildendes Polymer,
- mindestens einen up-conversion Leuchtstoff,
- optional mindestens ein Additiv,
- optional mindestens einen Härter,
wobei der Leuchtstoff ausgewählt ist aus der idealisierten allgemeinen Formel (I)
A_{1-x-y-z}B*_{y}B₂SiO₄:Ln¹ₓ,Ln²_{z}, I
mit
x = 0,0001 - 0,05, z = 0 oder z = 0,0001 bis 0,3 und y = x + z,
A ausgewählt aus Mg, Ca, Sr und Ba,
B ausgewählt aus Li, Na, K, Rb und Cs,
B* ausgewählt aus Li, Na und K, wobei B gleich B* oder B ungleich B* ist, bevorzugt B und B* sind ungleich, und
Ln¹ ausgewählt aus Praseodym (Pr), Erbium (Er) und Neodym (Nd),
optional Ln² ausgewählt aus Gadolinium (Gd).

2. Zusammensetzung nach Anspruch 1, **dadurch gekennzeichnet, dass** der Leuchtstoff mit Praseodym dotiert ist.

3. Zusammensetzung nach einem der vorgenannten Ansprüche, **dadurch gekennzeichnet, dass** der Leuchtstoff mit Praseodym dotiert und mit Gadolinium co-dotiert ist.

4. Zusammensetzung nach einem der vorgenannten Ansprüche, **dadurch gekennzeichnet, dass** der Leuchtstoff eine erstarrte Schmelze aus kristallinen Silikaten oder aus kristallinen Silikaten dotiert mit Lanthanoid-Ionen, umfassend mindestens einen Alkalimetall Ion und mindestens einen Erdalkalimetall Ion, bevorzugt dass die kristalline Silikate mit Praseodym dotiert ist und optional co-dotiert mit Gadolinium, ist.

5. Zusammensetzung nach einem der vorgenannten Ansprüche, **dadurch gekennzeichnet, dass** der Leuchtstoff ausgewählt aus der idealisierten allgemeinen Formel (la)
A_{1-x-y-z}B*_{y}B₂SiO₄:Prₓ,Gd_{z}, **Ia**
mit A = Mg, Ca, Sr, Ba und B = Li, Na, K, Rb, Cs, und,
wobei in Formel Ia x = 0,0001 - 0,05, z = 0 oder z = 0,0001 bis 0,3 und y = x + z ist,
B* ausgewählt aus Li, Na und K, die zur Ladungsausgleich der Silikate dienen, wobei B gleich B* oder B ungleich B* ist, bevorzugt B und B* sind ungleich.

6. Zusammensetzung nach einem der vorgenannten Ansprüche, **dadurch gekennzeichnet, dass** der Leuchtstoff ausgewählt aus der allgemeinen Formel (II)
(Ca₁₋ₐSrₐ)_{1-2b}Ln_{b}Na_{b}Li₂SiO₄ **II**
wobei
a = 0,0001 bis 1, bevorzugt 0,0001 bis 0,1
b = 0,0001 bis 1, bevorzugt 0,0001 bis 0,1
und Ln = Lanthanoid-Ion ausgewählt aus Praseodym, Gadolinium, Erbium, Neodym und für die Co-Dotierung mindestens einen von denen, bevorzugt Gadolinium.

7. Zusammensetzung nach einem der vorgenannten Ansprüche, **dadurch gekennzeichnet, dass** der Leuchtstoff, der bei Bestrahlung mit elektromagnetischer Strahlung mit geringerer Energie und höherer Wellenlänge im Bereich von 2000 nm bis 400 nm, insbesondere im Bereich von 800 nm bis 400 nm, elektromagnetische Strahlung mit höherer Energie und kürzerer Wellenlänge im Bereich von 400 nm bis 100 nm, bevorzugt im Bereich von 300 nm bis 200 nm emittiert, wobei die Intensität des Emissionsmaximums der elektromagnetischen Strahlung mit höherer Energie und kürzerer Wellenlänge eine Intensität von mindestens 1 • 10³ counts/(mm²*s), bevorzugt höher als 1 • 10⁴ counts/(mm²*s), besonders bevorzugt höher als 1 • 10⁵ counts/(mm^{2*}s).

8. Zusammensetzung nach einem der vorgenannten Ansprüche, **dadurch gekennzeichnet, dass** der Leuchtstoff gemäße Formel (II) XRPD Signale im Bereich von 23° 2Θ bis 27° 2Θ und von 34° 2Θ bis 39.5° 2Θ aufweist.

9. Zusammensetzung nach einem der vorgenannten Ansprüche, **dadurch gekennzeichnet, dass** das filmbildende Polymer funktionelle Gruppen, vorzugsweise acide Wasserstoffe, enthält, welche mit einem isocyanathaltigen Härter oder mit einem Katalysator reaktiv sind.

10. Zusammensetzung nach einem der vorgenannten Ansprüche, **dadurch gekennzeichnet, dass** das filmbildende Polymer aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Asparticpolymer oder Polyesterpolymere ausgewählt ist, das mit einem isocyanathaltigen Härter reagiert.

11. Zusammensetzung nach einem der vorstehenden Ansprüche, **dadurch gekennzeichnet, dass** das filmbildende Polymer resonanzarm ist.

12. Zusammensetzung nach einem der vorstehenden Ansprüche, **dadurch gekennzeichnet, dass** die Transmission des filmbildenden Polymers bei mindestens 75%, bevorzugt mindestens 80% und besonders bevorzugt mindestens 85%, mittels eines UV/VIS-Zweistrahlspektrometers liegt.

13. Zusammensetzung nach einem der vorstehenden Ansprüche, **dadurch gekennzeichnet, dass** die Transmission mindestens 70%, bevorzugt mindestens 75% und besonders bevorzugt mindestens 80%, mittels eines UV/VIS-Zweistrahlspektrometers beträgt.

14. Zusammensetzung nach einem der vorgenannten Ansprüche, **dadurch gekennzeichnet, dass** die Leuchtstoffe eine durchschnittliche Partikelgröße von d50 = 0,1 - 100 µm, bevorzugt d50 = 1 - 50 µm, gemessen nach ISO 13320:2020 und USP 429 aufweisen.

15. Zusammensetzung nach einem der vorgenannten Ansprüche, **dadurch gekennzeichnet, dass** die Additive ausgewählt sind aus der Gruppe der Dispergiermittel, Rheologiehilfsmittel, Verlaufsmittel, Netzmittel, Entschäumer und UV-Stabilisierungsmittel.

16. Zusammensetzung nach einem der vorgenannten Ansprüche, **dadurch gekennzeichnet, dass** der Härter ausgewählt ist aus der Gruppe der aliphatischen oder cycloaliphatische Isocyanate.

17. Zusammensetzung nach einem der vorgenannten Ansprüche, **dadurch gekennzeichnet, dass** daraus hergestellte Beschichtungen eine antimikrobielle Wirkung gegen Bakterien, Hefen, Schimmelpilze, Algen, Parasiten und Viren aufweisen.

18. Zusammensetzung nach einem der vorgenannten Ansprüche, **dadurch gekennzeichnet, dass** daraus hergestellte Beschichtungen eine antimikrobielle Wirkung gegen
- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Pathogene Umweltorganismen, vorzugsweise gegen *Cryptosporidium parvum, Giardia lamblia,* Amöben (*Arcanthamoeba* spp., *Naegleria* spp.), *E. coli,* coliforme Bakterien, Fäkalstreptokokken, *Salmonella* spp., *Shigella* spp., *Leginonella* spec., *Pseudomonas aeruginosa, Mykobakteria* spp., enterale Viren (z.B. Polio- und Hepatitis-A Virus);
- Pathogene in Lebensmitteln, vorzugsweise gegen*Bacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen,
aufweisen.

19. Verwendung der Zusammensetzung nach einem der vorgenannten Ansprüche zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

20. Verwendung der Zusammensetzung nach einem der Ansprüche 1 bis 18 zur Herstellung von Beschichtungen mit anti-mikrobieller Eigenschaft.

21. Verwendung der Zusammensetzung nach einem der Ansprüche 1 bis 18 zur Beschichtung von Substraten in Hygieneeinrichtungen, Krankenhäusern und in der Lebensmittelindustrie.

22. Verfahren zur Bildung einer anti-mikrobiellen Beschichtung auf einem Substrat, umfassend das Aufbringen einer härtbaren filmbildenden Zusammensetzung auf das Substrat, umfassend:
(a) mindestens ein filmbildendes Polymer, das funktionelle Gruppen enthält, welche mit einem isocyanathaltigen Härter reaktiv sind, ggf. durch einen Katalysator katalysiert
(b) mindestens einen Leuchtstoff gemäß der Formel (II) und
(c) einen Härter, enthaltend isocyanatfunktionelle Gruppen.

23. Verfahren nach Anspruch 22, wobei das Substrat Metall, mineralische Substrate, zellulosehaltige Untergründe, Holz sowie deren Hybride formstabile Kunststoffe und/oder Duromere enthält.

24. Verfahren nach einem der Ansprüche 22 - 23, wobei eine Grundierzusammensetzung auf das Substrat vor der Auftragung der härtbaren filmbildenden Zusammensetzung aufgetragen wird.

25. Gegenstand, **dadurch gekennzeichnet, dass** er mindestens teilweise, vorzugsweise vollständig, mit der härtbaren Zusammensetzung nach einem der Ansprüche 1 bis 18 beschichtet ist.
